# FASCICULE DE BREVET EUROPEEN

(11) **EP 0 935 484 B1**
(45) Date de publication et mention de la délivrance du brevet: **23.03.2005**
(21) Numéro de dépôt: 98939459.8
(22) Date de dépôt: 28.08.1998
(51) Int. Cl.: A61N 5/06

(54) **DISPOSITIF POUR L'IRRADIATION DE CAVITES INTERIEURES DE L'ORGANISME**
VORRICHTUNG ZUR BESTRAHLUNG DER INNENRÄUME DES ORGANISMUS
DEVICE FOR IRRADIATING INTERNAL CAVITIES OF THE BODY

(30) Priorité: 04.09.1997 FR 9711253
(43) Date de publication de la demande: 18.08.1999
(73) Titulaire: Medlight S.A., 1024 Ecublens (CH)
(72) Inventeur: BAYS, Roland, CH-1680 Romont (CH); WOODTLI, Alain, CH-2024 Saint-Aubin (CH); WAGNIERES, Georges, CH-1110 Morges (CH); VAN DEN BERGH, Hubert, CH-1376 Goumoens-la-Ville (CH)
(74) Mandataire: Besse, François
(86) Numéro de dépôt international: PCT/CH1998/000370
(87) Numéro de publication internationale: WO 1999/011322

(56) Documents cités:
- EP-A- 0 292 695
- EP-A- 0 411 132
- EP-A- 0 673 627
- WO-A-95/08949
- DE-A- 3 909 843
- FR-A- 2 600 205

## Description

### Domaine technique

La présente invention concerne un dispositif pour l'irradiation dé cavités intérieures de l'organisme, en particulier pour le traitement de certaines affections par la thérapie photodynamique, ce dispositif comprenant un cathéter souple, réalisé en un matériau transparent, contenant une fibre optique dont un tronçon d'extrémité est agencé pour diffuser radialement de la lumière provenant d'une source ménagée à l'autre extrémité de ladite fibre, et un ballonnet gonflable réalisé en un matériau élastomère ayant la propriété de diffuser la lumière et fixé à une extrémité dudit cathéter de façon à recouvrir le tronçon diffusant de la fibre optique.

### Technique antérieure

Pour le traitement de certaines lésions cancéreuses situées dans des cavités du corps humain telles que par exemple les bronches, l'oesophage ou l'utérus, on a de plus en plus souvent recours à la thérapie photodynamique qui implique une irradiation de la tumeur au moyen d'une lumière diffuse. Cette irradiation se fait à l'aide de dispositifs comportant un cathéter renfermant une fibre optique reliée à une source de lumière laser, qui peut être introduit dans le canal de biopsie d'un endoscope classique. Afin d'obtenir une diffusion de lumière la plus homogène possible et de ne pas endommager les parois de la cavité, on coiffe l'extrémité du cathéter introduite dans la cavité d'un ballon qui, lorsqu'il est mis en place est gonflé de manière à prendre appui sur les parois de la cavité à traiter.

Dans les dispositifs actuellement utilisés, notamment celui qui fait l'objet de la publication DE 39 09 843, lorsqu'on se trouve en présence d'une cavité irrégulière, pour obtenir l'homogénéité de la répartition lumineuse, on applique sur les parois de la cavité une couche d'un matériau ayant des propriétés diffusantes. Une enveloppe élastique peut également être utilisée. La pulvérisation de la couche diffusante est une opération délicate et si l'on utilise une enveloppe élastique gonflable dans ce genre de cavité, d'une part l'on est obligé d'appliquer une pression de gonflage assez élevée pour que l'enveloppe plaque le plus possible la paroi de la cavité, ce qui aboutit à des contraintes de pression élevées et différenciées sur les parois de la cavité et risque de modifier la vascularisation des tissus et, d'autre part, l'on crée des différences d'épaisseur de la paroi de l'enveloppe, ce qui aboutit à une transmission irrégulière de la lumière.

Un dispositif selon le préambule de la revendication 1 est divulgué dans le document FR 2 600 205.

### Exposé de l'invention

La présente invention a pour but de remédier à ces inconvénients en offrant un dispositif dans lequel l'enveloppe élastique, ou ballonnet, plaque bien contre la paroi à traiter et applique ainsi une pression uniforme sur les parois de la cavité, l'épaisseur des parois de ce ballonnet restant constante après le gonflage. De plus, la pression nécessaire audit gonflage est faible, ce qui fait que la pression résultante appliquée par ledit ballonnet sur les tissus de la cavité à traiter est également faible et ne risque pas de les endommager. De cette façon, on obtient une répartition homogène de la lumière diffusée dans les cavités à traiter de toutes formes tout en ménageant les parois desdites cavités.

Le ballonnet du dispositif de la présente invention est préfabriqué au moyen d'un moule dont la forme est sensiblement celle d'une cavité à traiter, de manière à en épouser la forme et à appliquer une pression uniforme sur les parois de ladite cavité.

De façon avantageuse, le ballonnet peut être fabriqué par moulage d'une empreinte spécifique de la cavité à traiter.

Dans toutes les formes de réalisation, le ballonnet comporte un col agencé pour être enfilé sur l'extrémité sur le cathéter entourant le tronçon diffusant de la fibre optique et, de ce fait, l'axe longitudinal du tronçon diffusant de la fibre optique est confondu avec l'axe de symétrie du ballonnet.

De préférence, la fibre optique est logée dans une gaine de protection.

Selon une forme de réalisation préférée, le cathéter comporte, à son extrémité opposée à celle recouverte par le ballonnet, une ouverture axiale agencée pour recevoir la fibre optique et une ouverture radiale agencée pour introduire les moyens de gonflage du ballonnet dans l'espace délimité par la paroi intérieure du cathéter et la gaine de protection renfermant la fibre optique.

Les moyens de gonflage du ballonnet peuvent être de l'air ou un liquide stérile, par exemple de l'eau.

Pour diffuser le moyen de gonflage dans ledit ballonnet, l'extrémité du cathéter recouverte par le ballonnet comporte des ouvertures radiales, régulièrement espacées, disposées sur sa périphérie.

Dans toutes les formes de réalisation, le ballonnet est agencé pour être gonflé lorsqu'il a été introduit dans la cavité à traiter.

D'une façon avantageuse, il peut comporter des moyens pour contrôler la pression appliquée par le ballonnet sur les parois de la cavité à traiter.

### Description sommaire des dessins

La présente invention sera mieux en référence à la description d'exemples de réalisation préférés et des dessins annexés dans lesquels :
la figure 1 représente une première forme de réalisation du dispositif selon l'invention destiné a irradier les bronches d'un patient, et
la figure 2 représente une seconde forme de réalisation du dispositif selon l'invention destiné à irradier l'utérus d'une patiente.

### Meilleures manières de réaliser l'invention

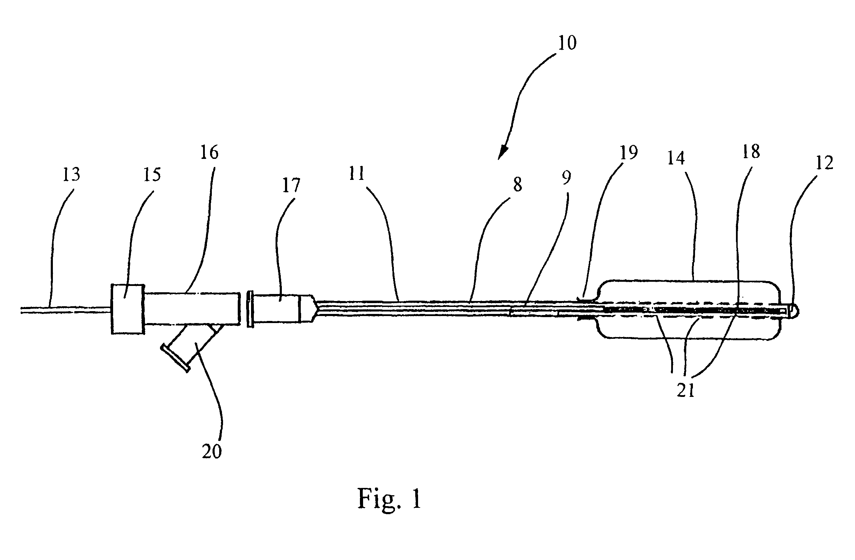

En référence à la figure 1, le dispositif 10, destiné à être un introduit dans les bronches d'un patient, se compose d'un cathéter 11 réalisé en un matériau souple transparent et fermé à l'une 12 de ses extrémités, d'une fibre optique 13 logée dans ledit cathéter et d'un ballonnet 14 gonflable fixé sur l'extrémité 12 du cathéter 11. Les dimensions du dispositif 10 sont telles qu'il peut être placé dans le canal de biopsie d'un endoscope classique (non représenté) destiné à l'exploration des organes du corps humain.

La fibre 13 dont le diamètre est inférieur à celui du cathéter 11, qui est revêtue d'une gaine de protection 9, est introduite dans le cathéter par une ouverture axiale 15 d'un embout 16 solidaire de l'autre extrémité 17 dudit cathéter jusqu'à ce qu'elle soit en contact avec l'extrémité 12 en définissant un espace 8 entre la paroi extérieure de la gaine 9 et la paroi intérieure du cathéter 11. Cette fibre 13, qui est agencée pour acheminer de la lumière issue d'une source (non représentée) à l'extrémité 12 du cathéter, est pourvue, à son extrémité introduite dans le cathéter, d'un tronçon 18 ayant des propriétés de diffusion radiale de la lumière transmise par ladite fibre.

Le ballonnet 14, qui est destiné à homogénéiser la lumière diffusée dans la cavité à traiter par le tronçon 18 de la fibre 13 afin que l'irradiation de ladite cavité, dans cet exemple les bronches, se fasse de façon uniforme, est un ballon gonflable qui, lorsqu'il est gonflé, doit se plaquer contre la paroi de la cavité à traiter. A cet effet il est réalisé en un matériau élastomère déformable tel que du latex ou du silicone qui, dans cette application, est chargé de particules d'une matière diffusante telle que le dioxyde de titane, pour améliorer les propriétés de diffusion dudit ballonnet. Ce ballonnet est enfilé plié sur l'extrémité 12 du cathéter de manière à recouvrir le tronçon diffusant 18. A cet effet, il est pourvu, à une de ses extrémités, d'un col 19 dont le diamètre est égal au diamètre extérieur du cathéter 11 afin de garantir le centrage du tronçon diffusant 18 de la fibre 13 par rapport au ballonnet 14. Il est fixé au cathéter par collage et ligature de son col 19 sur la paroi extérieure du cathéter 11.

Pour que la lumière irradie de façon uniforme la cavité à travers le ballonnet gonflable, il faut que ce dernier prenne la forme de la cavité tout en gardant une épaisseur de paroi constante afin que tous les points à irradier reçoivent la même quantité de lumière. Il n'est pas possible d'obtenir cela avec des ballonnets de forme standard allongée ou cylindrique. En conséquence, le ballonnet 14 de l'invention est réalisé directement à la forme de la cavité, dans cet exemple cylindrique allongée, dans laquelle il doit être introduit. Pour cela on procède, d'une manière connue en soi, à un moulage d'une empreinte spécifique de la cavité à traiter.

Le ballonnet, qui est introduit plié dans la cavité, est ensuite gonflé au moyen d'air ou d'un liquide stérile tel que de l'eau sous pression introduit dans l'espace 8 existant entre la fibre 13 et la paroi intérieure du cathéter, par une ouverture radiale 20 ménagée sur l'embout 16. Cet air ou ce liquide est injecté dans le ballonnet 14 par des ouvertures radiales 21, régulièrement espacées, disposées sur la périphérie du tronçon d'extrémité du cathéter se trouvant à l'intérieur du ballonnet. L'utilisation d'un liquide comme moyen de gonflage permet, si nécessaire, de refroidir le tronçon diffusant 18 de la fibre optique.

Etant donné que le ballonnet a la forme de la cavité, la pression nécessaire à son gonflage est faible et cela élimine les problèmes de changement des conditions de vascularisation des tissus concernés dues à une pression trop forte appliquées sur les parois de la cavité, ce qui produirait des variations dans l'efficacité de la thérapie.

Afin d'assurer l'étanchéité du dispositif les ouvertures 15 et 20 de l'embout 16 sont pourvues respectivement d'un bouchon de fermeture sous la forme d'un joint O-ring pour l'ouverture axiale 15 et d'un bouchon solidaire d'une vanne (non représentée), pour l'ouverture 21, ladite vanne permettant de réguler la pression sous laquelle le moyen de gonflage du ballonnet est introduit et ainsi de contrôler la pression résultante appliquée par le ballonnet aux tissus.

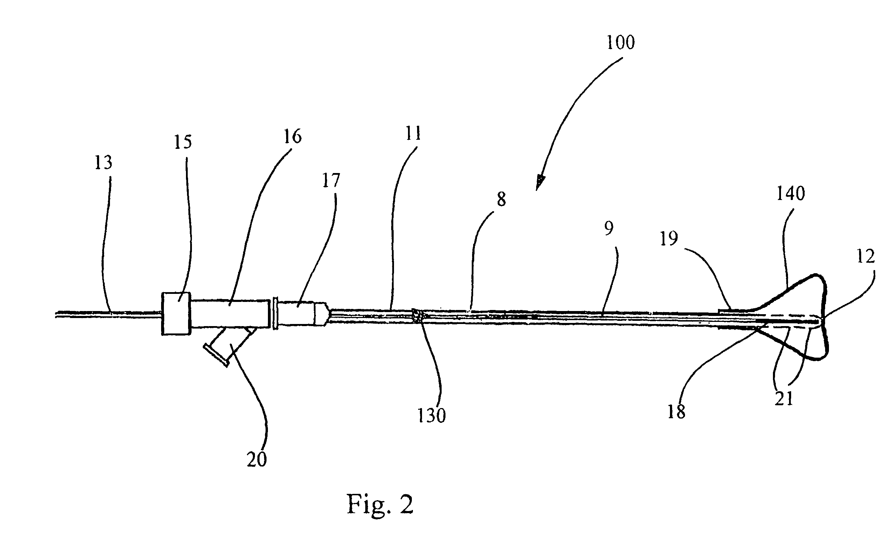

La figure 2 illustre un dispositif 100 destiné à traiter les parois de l'utérus d'une patiente. Les éléments qui le composent sont identiques à ceux décrits en référence à la figure 1 et portent la même référence, seul le ballonnet 140 étant différent. Ce ballonnet 140, qui a la forme d'un utérus, est obtenu par moulage d'un moule, par exemple en aluminium, réalisé à partir d'une empreinte de l'utérus à traiter. La flexibilité du ballonnet permet au dispositif de s'adapter aux dimensions réelles de l'utérus qui peuvent varier entre deux patientes et, par conséquent, il n'est pas nécessaire de réaliser un ballonnet par patiente à traiter.

Dans cette réalisation, le ballonnet présentant une forme particulière, il est nécessaire de prévoir sur le cathéter 11 un repère d'orientation 130 afin que, lors de l'introduction du dispositif dans le corps, le ballonnet 140 soit correctement positionné par rapport à l'utérus et qu'il se plaque bien contre la paroi à traiter lors de son gonflage.

Dans d'autres formes de réalisation, on peut prévoir le remplacement de la fibre optique unique par plusieurs fibres suivant l'éclairement requis.

## Revendications

1. Dispositif pour l'irradiation de cavités intérieures de l'organisme, en particulier pour le traitement de certaines affections par la thérapie photodynamique, ce dispositif comprenant un cathéter souple (11), réalisé en un matériau transparent, fermé à une (12) de ses extrémités, contenant une fibre optique (13) dont un tronçon d'extrémité (18) est agencé pour diffuser radialement de la lumière provenant d'une source ménagée à l'autre extrémité de ladite fibre, et un ballonnet gonflable (14, 140) réalisé en un matériau élastomère ayant la propriété de diffuser la lumière et fixé à une extrémité dudit cathéter de façon à recouvrir le tronçon diffusant (18) de la fibre optique (13), **caractérisé en ce que** ledit ballonnet (14, 140) est préfabriqué au moyen d'un moule dont la forme est sensiblement celle d'une cavité à traiter de manière, qu'une fois le ballonnet introduit dans ladite cavité et gonflé, il en épouse la forme et applique une pression uniforme sur ses parois.

2. Dispositif selon la revendication 1, **caractérisé en ce que** la forme du ballonnet (14, 140) obtenue par moulage est identique à celle d'une empreinte spécifique de ladite cavité.

3. Dispositif selon la revendication 1, **caractérisé en ce que** le ballonnet comporte un col (19) agencé pour être enfilé sur l'extrémité (12) du cathéter entourant le tronçon diffusant (18) de la fibre optique (13).

4. Dispositif selon la revendication 3, **caractérisé en ce que** l'axe longitudinal du tronçon diffusant (18) de la fibre optique (13) est confondu avec l'axe de symétrie du ballonnet (14, 140).

5. Dispositif selon la revendication 1, **caractérisé en ce que** la fibre optique (13) est logée dans une gaine de protection (9).

6. Dispositif selon les revendications 1 et 5, **caractérisé en ce que** le cathéter (11) comporte, à son extrémité (17) opposée à celle recouverte par le ballonnet (14, 140), une ouverture axiale (15) agencée pour recevoir la fibre optique (13) et une ouverture radiale (20) agencée pour introduire les moyens de gonflage du ballonnet (14, 140) dans l'espace délimité (8) par la paroi intérieure du cathéter et la gaine de protection (9) renfermant la fibre optique (13).

7. Dispositif selon la revendication 6, **caractérisé en ce que** les moyens de gonflage du ballonnet sont de l'air.

8. Dispositif selon la revendication 6, **caractérisé en ce que** les moyens de gonflage du ballonnet sont un liquide stérile.

9. Dispositif selon la revendication 6, **caractérisé en ce que** l'extrémité (12) du cathéter recouverte par le ballonnet (14, 140) comporte des ouvertures radiales (21) régulièrement espacées agencées pour diffuser le moyen de gonflage dans ledit ballonnet.

10. Dispositif selon la revendication 1, **caractérisé en ce que** le ballonnet (14, 140) est agencé pour être gonflé lorsqu'il a été introduit dans la cavité à traiter.

11. Dispositif selon la revendication 1, **caractérisé en ce qu'**il comporte des moyens pour contrôler la pression appliquée par le ballonnet (14, 140) sur les parois de la cavité à traiter.

## Patentansprüche

1. Vorrichtung zur Bestrahlung innerer Höhlungen des Organismus, insbesondere zur Behandlung bestimmter Störungen mittels photodynamischer Therapie, wobei die Vorrichtung einen biegsamen, aus einem transparenten Material hergestellten Katheter (11), der an einem (12) seiner Enden verschlossen ist und eine optische Faser (13) enthält, bei der ein Teilstück des Endes (18) zum radialen Diffundieren von Licht eingerichtet ist, das von einer am anderen Ende der Faser angebrachten Quelle stammt, und einen aufblasbaren, aus einem Elastomermaterial hergestellten Ballon (14, 140) umfasst, der die Eigenschaft besitzt, das Licht zu diffundieren, und an einem Ende des Katheters derart befestigt, dass er das diffundierende Teilstück (18) der optischen Faser (13) bedeckt, **dadurch gekennzeichnet, dass** der Ballon (14, 140) mithilfe einer Form, die im Wesentlichen die Gestalt der zu behandelnden Höhlung hat, derart vorgefertigt wird, dass, wenn der in die Höhlung eingeführte Ballon aufgeblasen wird, er sich an ihre Form anpasst und einen gleichmäßigen Druck auf die Wände ausübt.

2. Vorrichtung nach Anspruch 1, **dadurch gekennzeichnet, dass** die durch Abformung erhaltene Gestalt des Ballons (14, 140) identisch mit derjenigen eines spezifischen Abdrucks der Höhlung ist.

3. Vorrichtung nach Anspruch 1, **dadurch gekennzeichnet, dass** der Ballon einen Hals (19) umfasst, der zum Einfädeln in das Ende (12) des Katheters, welches das diffundierende Teilstück (18) der optischen Faser (13) umgibt, eingerichtet ist.

4. Vorrichtung nach Anspruch 3, **dadurch gekennzeichnet, dass** die Längsachse des diffundierenden Teilstücks (18) der optischen Faser (13) mit der Symmetrieachse des Ballons (14, 140) in Übereinstimmung gebracht worden ist.

5. Vorrichtung nach Anspruch 1, **dadurch gekennzeichnet, dass** die optische Faser (13) in einer Schutzhülle (9) untergebracht ist.

6. Vorrichtung nach den Ansprüchen 1 und 5, **dadurch gekennzeichnet, dass** der Katheter (11) an seinem Ende (17), das dem durch den Ballon (14, 140) bedeckten gegenüberliegt, eine axiale Öffnung (15), die zum Aufnehmen der optischen Faser (13) eingerichtet ist, und eine radiale Öffnung (20) umfasst, die zum Einbringen der Mittel zum Aufblasen des Ballons (14, 140) in den Raum (8), der durch die Innenwand des Katheters und die Schutzhülle (9), welche die optische Faser (13) umgibt, begrenzt wird, eingerichtet ist.

7. Vorrichtung nach Anspruch 6, **dadurch gekennzeichnet, dass** es sich bei den Mitteln zum Aufblasen des Ballons um Luft handelt.

8. Vorrichtung nach Anspruch 6, **dadurch gekennzeichnet, dass** es sich bei den Mitteln zum Aufblasen des Ballons um eine sterile Flüssigkeit handelt.

9. Vorrichtung nach Anspruch 6, **dadurch gekennzeichnet, dass** das Ende (12) des Katheters, das durch den Ballon (14, 140) bedeckt ist, radiale Öffnungen (21) umfasst, die in regelmäßigen Abständen angeordnet sind und zum Verteilen des Mittels zum Aufblasen in dem Ballon eingerichtet sind.

10. Vorrichtung nach Anspruch 1, **dadurch gekennzeichnet, dass** der Ballon (14, 140) derart eingerichtet ist, dass er aufgeblasen wird, wenn er in die zu behandelnden Höhlung eingebracht worden ist.

11. Vorrichtung nach Anspruch 1, **dadurch gekennzeichnet, dass** sie Mittel zum Kontrollieren des Drucks umfasst, der durch den Ballon (14, 140) auf die Wände der zu behandelnden Höhlung ausgeübt wird.

## Claims

1. Device for irradiating internal cavities of the body, in particular for treating certain diseases by photodynamic therapy, this device comprising a flexible catheter (11) made of a transparent material, closed at one (12) of its ends and containing a fibre optic (13) of which an end section (18) is designed to radially diffuse light coming from a source provided at the other end of said fibre optic, and an inflatable balloon (14, 140) made of an elastomeric material having the property of diffusing light and fixed on one end of said catheter in such a way as to cover the diffusing section (18) of the fibre optic (13), **characterized in that** said balloon (14, 140) is pre-fabricated using a mould whose shape is substantially that of a cavity to be treated, so that, once the balloon has been introduced into said cavity and inflated, it matches the shape thereof and applies uniform pressure on the walls of the cavity.

2. Device according to Claim 1, **characterized in that** the shape of the balloon (14, 140) obtained by moulding is identical to that of a specific impression of said cavity.

3. Device according to Claim 1, **characterized in that** the balloon has a neck (19) designed to be fitted on the end (12) of the catheter surrounding the diffusing section (18) of the fibre optic (13).

4. Device according to Claim 3, **characterized in that** the longitudinal axis of the diffusing section (18) of the fibre optic (13) coincides with the axis of symmetry of the balloon (14, 140).

5. Device according to Claim 1, **characterized in that** the fibre optic (13) is housed in a protective sheath (9).

6. Device according to Claims 1 and 5, **characterized in that** the catheter (11), at its end (17) remote from the end covered by the balloon (14, 140), comprises an axial aperture (15) designed to receive the fibre optic (13) and a radial aperture (20) designed for introducing the means of inflating the balloon (14, 140) into the space (8) delimited by the inner wall of the catheter and the protective sheath (9) enclosing the fibre optic (13).

7. Device according to Claim 6, **characterized in that** the means of inflating the balloon is air.

8. Device according to Claim 6, **characterized in that** the means of inflating the balloon is a sterile liquid.

9. Device according to Claim 6, **characterized in that** the end (12) of the catheter covered by the balloon (14, 140) comprises radial apertures (21) spaced at regular intervals and designed to distribute the means of inflation in said balloon.

10. Device according to Claim 1, **characterized in that** the balloon (14, 140) is designed to be inflated when it has been introduced into the cavity to be treated.

11. Device according to Claim 1, **characterized in that** it comprises means for controlling the pressure applied by the balloon (14, 140) on the walls of the cavity to be treated.
